# EUROPEAN PATENT APPLICATION

(11) **EP 4 234 008 A1**
(43) Date of publication of application: **30.08.2023**
(21) Application number: 21882531.3
(22) Date of filing: 30.09.2021
(51) Int. Cl.: A61N 5/06

(54) **LIGHT EMISSION UNIT AND LIGHT EMISSION TYPE COSMETIC DEVICE**

(30) Priority: 22.10.2020 JP 2020177498
(71) Applicant: Panasonic Intellectual Property Management Co., Ltd., Osaka-shi, Osaka 540-6207 (JP)
(72) Inventor: KANEKO, Shota, Osaka-shi, Osaka 540-6207 (JP)
(74) Representative: Grünecker Patent- und Rechtsanwälte PartG mbB
(86) International application number: PCT/JP2021/036121
(87) International publication number: WO 2022/085390

(57) **Abstract**

Light-emitting unit (30) includes first light source unit (32) having a predetermined wavelength spectrum; second light source unit (33) having a wavelength spectrum different from that of first light source unit (32); and output aperture (31) outputting at least part of the electromagnetic waves emitted from first light source unit (32) and at least part of the electromagnetic waves emitted from second light source unit (33), when first light source unit (32) and second light source unit (33) are caused to emit their respective electromagnetic waves. First light source unit (32) is disposed at a position reducing interference with the electromagnetic waves emitted from second light source unit (33), and second light source unit (33) is disposed at a position reducing interference with the electromagnetic waves emitted from first light source unit (32).

## Description

### TECHNICAL FIELD

The present disclosure relates to a light-emitting unit and a light-emitting beauty device.

### BACKGROUND ART

As a means for achieving aesthetic effects such as beautifying skin or inhibiting unwanted hair, a method of irradiating a skin surface of a subject with light has been known. As a device for achieving aesthetic effects by irradiating a skin surface of a subject with light, a light-emitting beauty device has been known.

Such a light-emitting beauty device includes a light-emitting unit including a light source. As the light source of such a light-emitting beauty device, a light source having a narrow-band wavelength spectrum, such as a light-emitting diode (LED) or a laser, or a light source having a broad-band wavelength spectrum such as a flash lamp is generally used.

It has been known that the aesthetic effects achieved by irradiating skin with light emitted from a light source differ depending on the wavelength spectrum. Therefore, in recent years, there has been a need for capability for irradiating the skin with light emitted from light sources having different types of wavelength spectra, respectively, at the same time, so that different aesthetic effects are achieved.

Note that the light described above is the light in a broad sense (electromagnetic waves), and includes not only visible light but also light having ultra-violet and infrared wavelengths.

As a light-emitting beauty device capable of achieving various aesthetic effects, a light-emitting beauty devices disclosed in PTL 1 and PTL 2 below have been developed.

### Citation List

### Patent Literatures

PTL 1: Japanese Translation of PCT International Application Publication No. 2009-532079
PTL 2: Japanese Patent No. 6296743

### SUMMARY OF THE INVENTION

In the light-emitting beauty device disclosed in PTL 1, different types of light sources are disposed on the same plane so that the aesthetic effects of the light at a plurality of respective wavelength spectra are achieved at the same time.

Furthermore, in the light-emitting beauty device disclosed in PTL 2, with the output aperture of a flash lamp at the center, by disposing separate light sources, such as LEDs, around the output aperture, light emission at a plurality of wavelength spectra can achieved on the same plane.

However, in the configurations disclosed in PTL 1 and PTL 2, the area of the skin irradiated with the light becomes inevitably large. Hence, a small area such as a nostril or the outer corner of an eye can only be irradiated with the light of the light sources that are disposed around the output aperture.

As described above, with the conventional technologies, there are many body parts where light emitted from a plurality of light sources cannot be applied to the same spot, and it has been difficult to enhance the aesthetic effects, disadvantageously.

Therefore, an object of the present disclosure is to achieve a light-emitting unit and a light-emitting beauty device capable of further enhancing aesthetic effects.

A light-emitting unit according to one aspect of the present disclosure includes: a first light source unit having a predetermined wavelength spectrum; a second light source unit having a wavelength spectrum different from the predetermined wavelength spectrum of the first light source unit; and an output aperture through which at least part of a first electromagnetic wave emitted from the first light source unit and at least part of a second electromagnetic wave emitted from the second light source unit are outputted when the first light source unit and the second light source unit are caused to emit the first electromagnetic wave and the second electromagnetic wave, respectively. The first light source unit is disposed at a position reducing interference with the second electromagnetic wave emitted from the second light source unit, and the second light source unit is disposed at a position reducing interference with the first electromagnetic wave emitted from the first light source unit.

A light-emitting beauty device according to another aspect of the present disclosure is a device including the light-emitting unit according to the aspect described above.

### BRIEF DESCRIPTION OF THE DRAWINGS

Fig. 1 is a perspective view illustrating a light-emitting beauty device according to an exemplary embodiment.
Fig. 2 is a front view illustrating an attachment incorporating the light-emitting unit according to the exemplary embodiment.
Fig. 3 is a side view illustrating the attachment incorporating the light-emitting unit according to the exemplary embodiment.
Fig. 4 is a transverse cross-sectional view illustrating the attachment incorporating the light-emitting unit according to the exemplary embodiment.
Fig. 5 is a side sectional view illustrating the attachment incorporating the light-emitting unit according to the exemplary embodiment.
Fig. 6 is a perspective view illustrating the attachment incorporating the light-emitting unit according to the exemplary embodiment.
Fig. 7 is a perspective view illustrating a first light source unit and a second light source unit included in the light-emitting unit according to the exemplary embodiment.
Fig. 8 is a block diagram illustrating a functional configuration of the light-emitting beauty device according to the exemplary embodiment.

### DESCRIPTION OF EMBODIMENT

Hereinafter, an exemplary embodiment will be described in detail with reference to the drawings. However, detailed descriptions more than necessary may be omitted. For example, the detailed descriptions of already well-known matters and redundant explanations of substantially the same elements are sometimes omitted.

Note that, the accompanying drawings and the following descriptions are only provided to facilitate those skilled in the art to fully understand the present disclosure, and are not intended to limit the subject matter as defined in the claims in any way.

In the following description, light in a broad sense, including not only visible light but also light having ultraviolet and infrared wavelengths, will be referred to as an electromagnetic wave.

Furthermore, in the explanation of the exemplary embodiment below, the vertical direction of the light-emitting beauty device having an output aperture thereof facing upward is defined as up-down direction Z; a longitudinal horizontal direction of the light-emitting beauty device is defined as width direction Y; and a short-side horizontal direction of the light-emitting beauty device (a direction orthogonal to up-down direction Z and width direction Y) is defined as front-back direction X.

In the present exemplary embodiment, a side of the main body provided with a switch is defined as a front side in the front-back direction.

### (Exemplary embodiment)

### [Example of configuration of light-emitting beauty device]

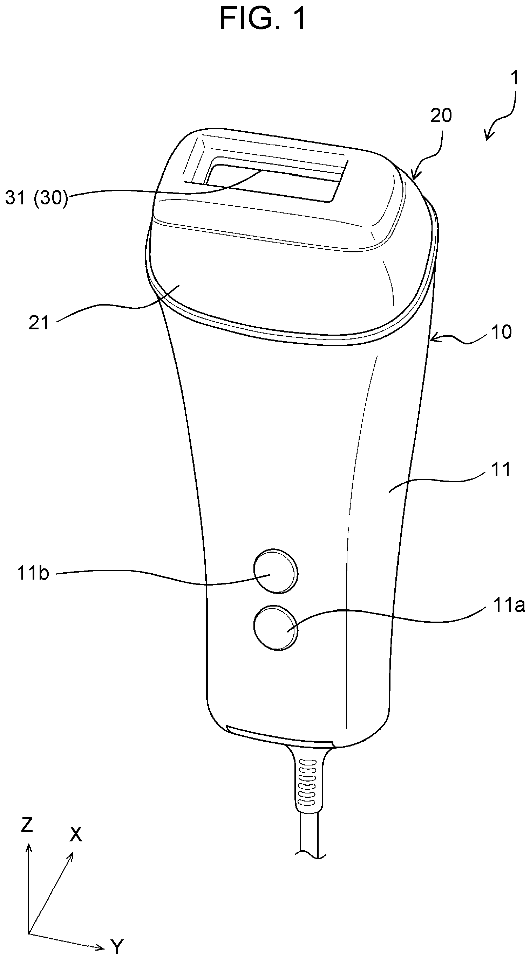

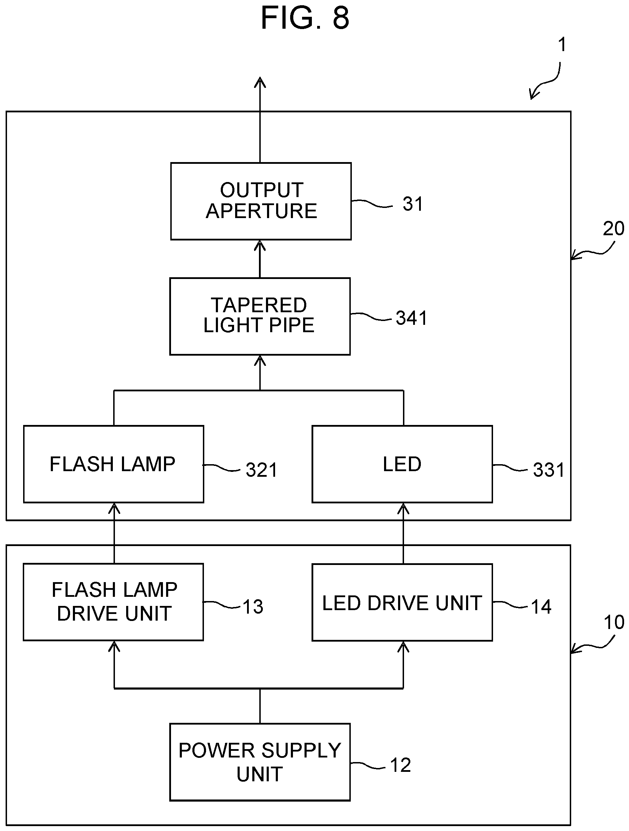

To begin with, one example of a configuration of the light-emitting beauty device will be described with reference to Figs. 1 and 8. Fig. 1 is a perspective view illustrating light-emitting beauty device 1 according to an exemplary embodiment. Fig. 8 is a block diagram illustrating a functional configuration of light-emitting beauty device 1 according to the exemplary embodiment.

As illustrated in Fig. 1, light-emitting beauty device 1 according to the present exemplary embodiment includes main body 10 and attachment 20 detachably attached to main body 10.

Main body 10 has an elongated shape in up-down direction Z, and has a size that allowing the user to hold with one hand. As described above, in the present exemplary embodiment, main body 10 functions as a grip that can be held by hand. Attachment (light emission attachment) 20 including light-emitting unit 30 for irradiating the skin of a subject with light is attached to an upper end of main body 10.

Light-emitting beauty device 1 to which light emission attachment (attachment 20) is attached is, for example, a device for enhancing the aesthetic effects given to the skin of the subject by irradiating the skin of the face or the limbs of the subject, with electromagnetic waves having a predetermined wavelength spectrum.

Note that attachment 20 may be one of a plurality of light emission attachments, such as an attachment for body and an attachment for face. In this manner, it is possible to use the plurality of attachments depending on applications, so that the aesthetic effects can be achieved more efficiently. In addition, an attachment having a function other than light emission, such as an attachment for hair removal, may also be provided.

In the present exemplary embodiment, main body 10 includes housing 11 made of a synthetic resin, and housing 11 can be formed by joining a plurality of separate parts, for example. There is a cavity inside housing 11, and various electric components are housed inside the cavity.

In the present exemplary embodiment, as illustrated in Fig. 8, units housed inside the cavity in housing 11 include flash lamp drive unit 13 that drives flash lamp (first light source) 321, which is to be described later, LED drive unit 14 that drives LED (second light source) 331, which is to be described later, and power supply unit 12 that supplies power to flash lamp drive unit 13 and LED drive unit 14.

In the present exemplary embodiment, housing 11 is also provided with push operation switch 11a for causing light-emitting beauty device 1 to operate (turning on and off the power supply). In the present exemplary embodiment, push operation switch 11a is exemplified as the switch, but a slide switch or another switch may be used, as long as the switch is capable of turning on and off the power.

Housing 11 is also provided with push selection switch 11b enabled to switch to drive any one or both of flash lamp drive unit 13 and LED drive unit 14. Selection switch 1 1b may also be a slide switch or any another switch.

### [Example of configuration of light-emitting unit]

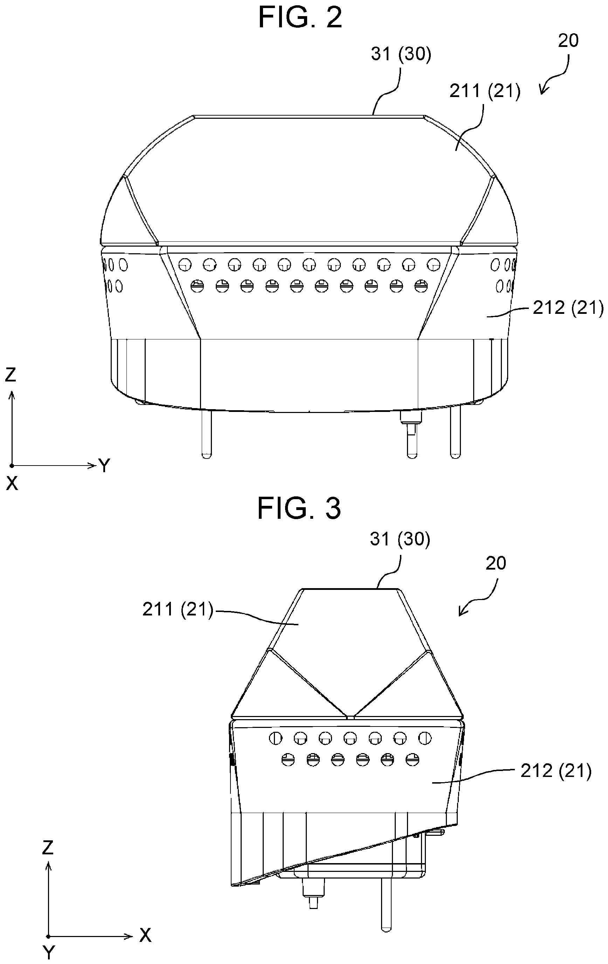

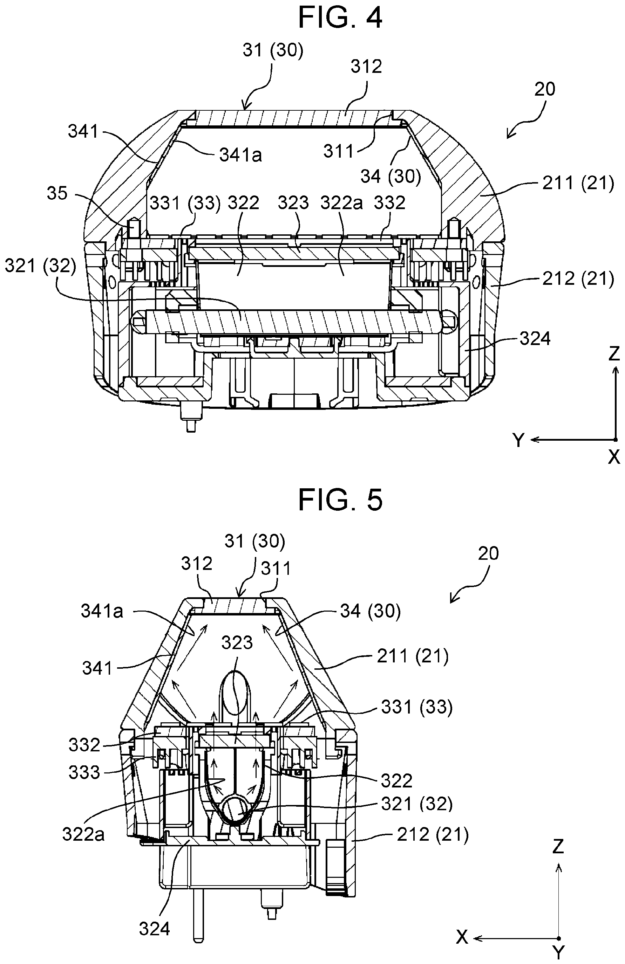

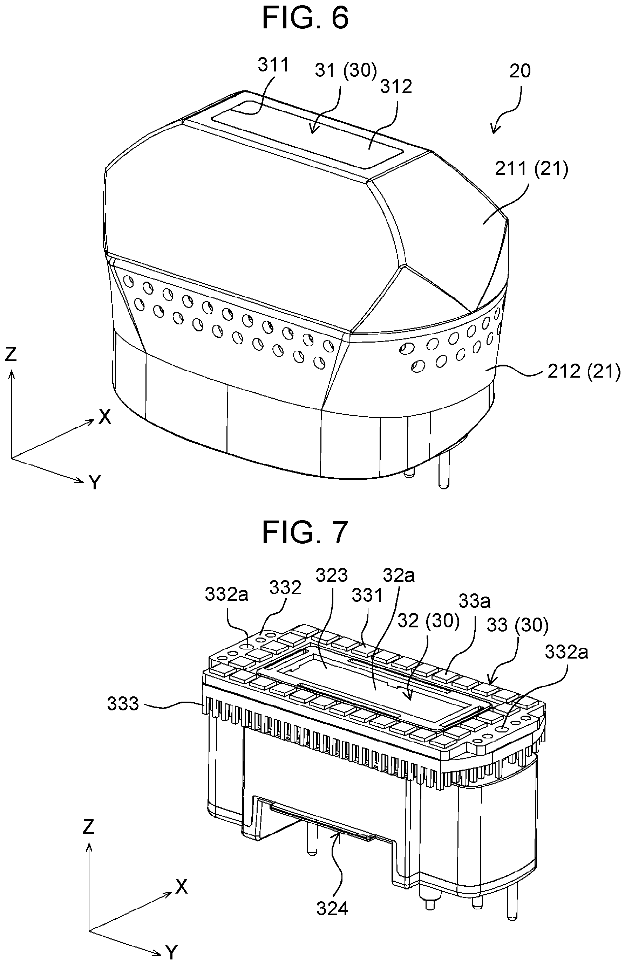

One example of a configuration of the light-emitting unit will be described with reference to Figs. 2 to 8. Figs. 2 and 3 are a front view and a side view, respectively, illustrating attachment 20 incorporating light-emitting unit 30 according to the exemplary embodiment. Figs. 4 and 5 are a transverse sectional view and a side sectional view, respectively, illustrating attachment 20 incorporating light-emitting unit 30. Fig. 6 is a perspective view illustrating attachment 20 incorporating light-emitting unit 30. Fig. 7 is a perspective view illustrating first light source unit 32 and second light source unit 33 of light-emitting unit 30 according to the exemplary embodiment.

Light-emitting unit 30 according to the present exemplary embodiment is incorporated inside (provided inside) attachment 20 described above.

Specifically, attachment 20 includes housing 21 making up an outer shell of attachment 20, and light-emitting unit 30 is incorporated inside housing 21.

Housing 21 is formed by joining upper housing 211 and lower housing 212 that are vertically separated parts, and a cavity vertically penetrating therethrough is formed inside housing 21 (see Figs. 4 and 5). Note that, in the present exemplary embodiment, an opening facing upward, among top and bottom openings of the cavity provided inside housing 21, serves as opening 311 of output aperture 31, which will be described later, and output aperture 31 is formed by inserting light-guide plate 312 into the opening.

Light-emitting unit 30 includes flash lamp unit (first light source unit) 32, LED unit (second light source unit) 33, and output aperture 31 located above flash lamp unit 32 and LED unit 33. The wavelength spectrum of the electromagnetic waves emitted from flash lamp unit 32 is different from the wavelength spectrum of the electromagnetic waves emitted from LED unit 33.

In the present exemplary embodiment, flash lamp 321 that is the first light source is used in flash lamp unit 32, and LEDs 331 that are the second light source is used in LED unit 33. Thus, the electromagnetic waves emitted from flash lamp unit 32 have a predetermined wavelength spectrum. The electromagnetic waves emitted from LED unit 33 have a predetermined wavelength spectrum different from that of the electromagnetic waves emitted from flash lamp unit 32.

When flash lamp 321 is used as the first light source for achieving aesthetic effects as in the present exemplary embodiment, broadband light across a range of 400 nm to 1200 nm is emitted. Therefore, not only a wide range of aesthetic effects but also a hair removal effect can be achieved on the skin of the subject.

In the present exemplary embodiment, flash lamp 321 having a substantially cylindrical shape (a flash lamp that is a cylindrical tube) is used, and flash lamp 321 that is a cylindrical tube is disposed in such a manner that the axial direction thereof extends along the width direction Y (see Figs. 4 and 5).

When cylindrical tubular flash lamp 321 is used in the manner described above, the electromagnetic waves (light) are emitted in the radial directions of the cylindrical tube. Therefore, the electromagnetic waves (lights) cannot be efficiently guided to output aperture 31, and a relatively large amount of electromagnetic wave (light) is wasted.

Therefore, in the present exemplary embodiment, flash lamp unit 32 includes reflection mirror 322 for guiding at least some of the electromagnetic waves emitted from flash lamp 321 and travelling in a direction other than the direction toward output aperture 31, to output aperture 31.

Reflection mirror 322 has a shape of resultant of bending a substantially rectangular plate-like member in a U shape, with the opening thereof upward in a side view. Mirror surface 322a is provided on the inner surface of reflection mirror 322. Cylindrical tubular flash lamp 321 is disposed facing mirror surface 322a, at the bottom of substantially U-shaped reflection mirror 322 in a side view. At this time, cylindrical tubular flash lamp 321 is preferably disposed at a focal point of a parabola approximating reflection mirror 322 having a substantially U-shape. In this manner, reflection mirror 322 can reflect the electromagnetic waves emitted from cylindrical tubular flash lamp 321 upward more reliably.

Furthermore, in the present exemplary embodiment, flash lamp filter 323 is disposed above cylindrical tubular flash lamp 321 and reflection mirror 322 having a substantially U-shape in a side view, and below output aperture 31 in up-down direction Z (on the side of the first light source). The electromagnetic waves emitted from cylindrical tubular flash lamp 321 and the electromagnetic waves reflected by mirror surface 322a of reflection mirror 322 pass through flash lamp filter 323, and head to output aperture 31. As described above, in the present exemplary embodiment, flash lamp filter 323 serves as an output aperture of flash lamp unit 32.

Flash lamp filter 323 may be formed of a material that easily transmits at least part of the electromagnetic waves emitted from at least flash lamp 321. Flash lamp filter 323 may also be formed by attaching a filter to the surface of a body made of a material such as glass or acrylic. At this time, it is possible to select a material transmitting the entire wavelengths. When a material transmitting of the entire wavelengths is selected, it is possible to take out higher energy. Flash lamp filter 323 may also function as an optical filter for removing light at a specific wavelength. For example, by removing light at 400 nm to 550 nm that is likely to cause pain when the skin is irradiated with electromagnetic waves (light) from flash lamp 321, light emission causing less pain can be achieved. Flash lamp filter 323 may also be configured as a bandpass filter to take out light at a specific wavelength, so that only the light giving the aesthetic effects can be applied to the skin.

In the present exemplary embodiment, holder casing 324 holds cylindrical tubular flash lamp 321, reflection mirror 322, and flash lamp filter 323 (see Figs. 4, 5, and 7).

As described above, in the present exemplary embodiment, while one flash lamp 321 is used as the first light source for providing the aesthetic effects, a plurality of LEDs 331 are used as the second light source for providing the aesthetic effects.

As the plurality of LEDs 331, surface-mounted LEDs are used, and the plurality of LEDs 331 are mounted on LED mount board 332. In the present exemplary embodiment, LED mount board 332 is disposed in a manner surrounding the periphery of the upper surface (flash lamp filter 323) of flash lamp unit 32, and the plurality of LEDs 331 are mounted on LED mount board 332 in a manner spaced from one another, at substantially equal intervals along the entire circumference. Therefore, in the present exemplary embodiment, the plurality of LEDs 331 are disposed in a manner surrounding the entire periphery of the upper surface of flash lamp unit 32 (flash lamp filter 323).

Any LEDs other than the surface-mounted LEDs may also be used as LEDs 331.

As in the present exemplary embodiment, by using LEDs 331 as the second light source for achieving the aesthetic effects, it is possible to select the wavelength depending on the aesthetic effects to be achieved. Therefore, not only desired aesthetic effects but also the hair removal effect can be achieved.

For example, by selecting LEDs 331 having the following wavelength band, it is possible to achieve the aesthetic effects described below. Note that the following wavelengths indicate peak wavelengths, and part of the electromagnetic waves (light) emitted from LEDs 331 may include wavelengths outside these range.
(1) 400 nm to 550 nm: fine wrinkle improvement, acne improvement, reddish face improvement, moisturization, pore improvement, anti-inflammatory action, sebum reduction, wound healing
(2) 550 nm to 620 nm: fine wrinkle improvement, nasolabial fold improvement, turnover promotion, dark spot improvement
(3) 620 nm to 750 nm: wound healing, immune activation, wrinkle improvement, dark spot improvement, turnover promotion, collagen production
(4)750 nm to 2000 nm: wound healing, immune activation, turnover promotion

When an LED having a wavelength spectrum of 550 nm to 1000 nm is used, an effect of improving skin brightness can be achieved.

In addition, LEDs having the same wavelength spectrum do not need to be used as the plurality of LEDs 331, and LEDs having different wavelength spectra may be used in combination. For example, by using an LED at 450 nm and an LED at 630 nm, effects of acne improvement and collagen production can be sought.

In addition, the wavelength spectrum of the electromagnetic waves emitted from LEDs 331 may be temperature dependent. As an example of such an LED, an LED emitting red light may be used. As described above, by using a temperature-dependent LED is used, various effects can be provided to the skin depending on a change in the temperature at the time of use. In other words, a plurality of effects can be provided to the skin using one LED.

In the present exemplary embodiment, the plurality of LEDs 331 are thermally connected to heat sink 333 via LED mount board 332, so that heat sink 333 dissipates the heat from the LEDs 331.

As described above, in the present exemplary embodiment, LED unit 33 includes the plurality of LEDs 331 that are the second light source, LED mount board 332 on which the plurality of LEDs 331 are mounted, and heat sink 333 that dissipates the heat of LEDs 331.

Furthermore, as illustrated in Fig. 7, flash lamp unit 32 and LED unit 33 integrated with each other are housed inside the space of housing 21.

Specifically, by inserting and fixing flash lamp filter 323 of flash lamp unit 32 into the central opening of LED mount board 332 having a substantially square (rectangular ring) shape in a plan view, holder casing 324 holding flash lamp 321, reflection mirror 322, and flash lamp filter 323 that is a cylindrical tube is integrated with LED unit 33.

As illustrated in Fig. 7, mount holes 332a are provided on both sides of LED mount board 332 in the width direction Y. By inserting screws 35 into respective mount holes 332a and fixing the screws onto the upper housing 211, flash lamp unit 32 and LED unit 33 are fixed to housing 21 (see Fig. 4).

From flash lamp unit 32, electromagnetic waves (light) are emitted upward in the up-down direction X (+Z direction). Because the plurality of LEDs 331 are top-emitting LEDs in which electromagnetic waves (light) are emitted upward in the up-down direction X (+ Z direction), it is not necessary to consider electromagnetic waves (light) emitted downward in the up-down direction X (-Z direction).

However, because LEDs 331 have a light distribution, the light spreads in the XY directions. Therefore, if no particular consideration is given, only small part of the electromagnetic waves (light) emitted from LEDs 331 is output from output aperture 31.

Therefore, in the present exemplary embodiment, light-emitting unit 30 includes an optical system disposed between flash lamp unit 32 and LED unit 33, and output aperture 31, and configured to guide at least part of the electromagnetic waves (light) emitted from the light source unit of at least one of flash lamp unit 32 and LED unit 33, to output aperture 31. As described above, light-emitting unit 30 includes the optical system as means for guiding the electromagnetic waves (light) emitted from LEDs 331 to output aperture 31, so that a larger amount electromagnetic waves (light) can be applied to the skin.

In addition, in order to prevent excessive damages of the skin or to achieve certain aesthetic effects, when the electromagnetic waves (light) is applied to the skin, the distribution of the electromagnetic waves (light) is preferably uniform in output aperture 31.

To achieve this end, in the present exemplary embodiment, integrator optical system 34 capable of reducing the brightness unevenness by mixing electromagnetic waves (light) is used as the optical system. As such integrator optical system 34, a conventionally known system may be used.

In the present exemplary embodiment, as an example of integrator optical system 34, light pipe 341 surrounded by mirror surface 341a capable of reflecting electromagnetic waves (light) in the wavelength spectrum emitted from flash lamp unit 32 and LED unit 33 is used.

Furthermore, in the present exemplary embodiment, as integrator optical system 34, integrator optical system 34 is configured as tapered light pipe 341 that is a combination of four mirrors, combined in such a manner that an incident side (on the side nearer to LEDs 331; on the lower side in the up-down direction Z) has an opening capable of housing all of the plurality of LEDs 331, and also in such a manner that the area of the opening decreases toward the output side (toward the top in the up-down direction Z). Therefore, in the present exemplary embodiment, tapered light pipe 341 has an internal space having a substantially quadrangular frustum shape and vertically penetrating therethrough.

With such tapered light pipe 341, integrator optical system 34 can be achieved more inexpensively, and the electromagnetic waves (light) from LEDs 331 can be guided to output aperture 31 more efficiently, and the electromagnetic waves (light) can be substantially uniformized in output aperture 31.

As described above, in the present exemplary embodiment, mirror surface 341a of light pipe 341 has four mirror surfaces inclined with respect to the central axis of light pipe 341.

At least one mirror surface among the four mirror surfaces may be a mirror surface extending in parallel with the central axis of light pipe 341.

However, even if integrator optical system 34 is used, depending on how flash lamp unit 32 and LEDs 331 (LED unit 33) are arranged, emission of the electromagnetic waves (light) from one toward output aperture 31 may be hindered by the light source unit of the other, and electromagnetic waves (light) emitted from output aperture 31 may become reduced.

Therefore, in the present exemplary embodiment, flash lamp unit 32 is disposed at a position interfering with less with the electromagnetic waves emitted from LED unit 33, and LED unit 33 is disposed at a position interfering with less with the electromagnetic waves emitted from flash lamp unit 32. As described above, in the present exemplary embodiment, by reducing the obstructions in the emissions of the electromagnetic waves (light) toward output aperture 31 more reliably, a sufficient amount of light can be taken out of output aperture 31.

Specifically, in the present exemplary embodiment, flash lamp unit 32 is disposed at a position no overlapping with the electromagnetic waves (light) emitted in a principal direction from LED unit 33, until the electromagnetic waves (light) emitted from LED unit 33 reach output aperture 31. Furthermore, LED unit 33 is disposed not overlapping with the electromagnetic waves (light) emitted in the principal direction from flash lamp unit 32, until the electromagnetic waves (light) emitted from flash lamp unit 32 reach output aperture 31. The principal direction of the electromagnetic waves (light) emitted from flash lamp unit 32 is up in the up-down direction Z, and the principal direction of the electromagnetic waves (light) emitted from LED unit 33 is up in the up-down direction Z.

Furthermore, in the present exemplary embodiment, as illustrated in Fig. 7, LED unit 33 is disposed not overlapping with flash lamp unit 32 in a plan view (in a view from output aperture 31), and flash lamp unit 32 and LED unit 33 are disposed in such a manner that light-emitting surface 32a of flash lamp unit 32 and light-emitting surface 33a of LED unit 33 are substantially on the same plane.

At this time, flash lamp unit 32 is disposed at a position not interfering with the electromagnetic waves within a half-value angle of the electromagnetic waves emitted from LED unit 33, and LED unit 33 is disposed at a position not interfering with the electromagnetic waves within a half-value angle of the electromagnetic waves emitted from flash lamp unit 32.

In this manner, electromagnetic waves (light) emitted from each of flash lamp unit 32 and LED unit 33 can be guided to output aperture 31 without interfering with the light source unit of the other.

Note that there might be cases in which it is not possible in reality to dispose the light sources exactly on the same plane. However, in such a case, each of the light source unit is preferably disposed at a position not blocking the light within a half-value angle of the electromagnetic waves (light) emitted from the other light source. With this, too, it becomes possible to take out a sufficient amount of light.

### [One example of operation of light-emitting beauty device]

An operation of light-emitting beauty device 1 including light-emitting unit 30 having a configuration described above will be explained.

To begin with, by attaching attachment 20 incorporating light-emitting unit 30 to main body 10, light-emitting beauty device 1 is made ready to use.

Operation switch 11a of light-emitting beauty device 1 ready to use is then operated to turn the power on.

At this time, when both of flash lamp drive unit 13 and LED drive unit 14 are selected to be driven, with the use of selection switch 11b, power is supplied from power supply unit 12 to flash lamp drive unit 13, so that flash lamp drive unit 13 causes flash lamp 321 to emit light. The power is also supplied from power supply unit 12 to LED drive unit 14, so that LED drive unit 14 causes LEDs 331 to emit light.

The electromagnetic waves (light) emitted from flash lamp 321 and the electromagnetic waves (light) emitted from LEDs 331 are then guided into integrator optical system 34, so that the two types of electromagnetic waves (light) having been more uniformly mixed are emitted outside via output aperture 31.

With emission of such light, light-emitting beauty device 1 is slid along the skin, with output aperture 31 being covered by the skin. In this manner, the skin surface of the subject is irradiated with light, and desired aesthetic effects are given to the skin of the subject.

As described above, in the present exemplary embodiment, when the electromagnetic waves (light) are emitted from flash lamp unit 32 and from LED unit 33, at least part of the respective two types of electromagnetic waves (light) is emitted from the same area (one output aperture 31). In the present exemplary embodiment, the two types of electromagnetic waves (light) are electromagnetic waves emitted from flash lamp unit 32 and electromagnetic waves emitted from LED unit 33.

In addition, when one of flash lamp drive unit 13 and LED drive unit 14 is selected to be driven using selection switch 11b at the time of power-on, the electromagnetic waves (light) emitted from the one light source unit are emitted from output aperture 31.

### [Actions and effects]

Hereinafter, characteristic configurations of the light-emitting unit and the light-emitting beauty device shown in the exemplary embodiment and effects achieved thereby will be described.

Light-emitting unit 30 described above in the exemplary embodiment includes first light source unit 32 having a predetermined wavelength spectrum; second light source unit 33 having a wavelength spectrum different from that of first light source unit 32; and output aperture 31 outputting at least part of the electromagnetic waves emitted from first light source unit 32 and at least part of the electromagnetic waves emitted from second light source unit 33, when first light source unit 32 and second light source unit 33 are caused to emit their respective electromagnetic waves. In addition, first light source unit 32 is disposed at a position reducing interference with the electromagnetic waves emitted from second light source unit 33, and second light source unit 33 is disposed at a position reducing interference with the electromagnetic waves emitted from first light source unit 32.

In this manner, light (electromagnetic waves) from light sources having respectively different wavelength spectra can be emitted via the same area (one output aperture). Therefore, it is possible to reduce a skin area that is only applied with the light (electromagnetic waves) from only one of the light sources having respectively different wavelength spectra. Therefore, it becomes possible to irradiate larger areas of the skin with light (electromagnetic waves) having different wavelength spectra at the same time, and it becomes possible to further enhance the aesthetic effects given to the subject.

In addition, even if the area of the output aperture is made small, it is possible to cause the plurality of light sources to emit light (electromagnetic wave) at the same time via the output aperture, so that the light (electromagnetic wave) emitted from the plurality of light sources can be applied to a small area of a nostril or the outer corner of an eye, at the same time. As a result, the aesthetic effects can be further enhanced.

In the manner described above, light-emitting unit 30 according to the present exemplary embodiment includes first light source unit 32 having a predetermined wavelength spectrum; second light source unit 33 having a wavelength spectrum different from that of first light source unit 32; and output aperture 31 outputting at least part of the electromagnetic wave emitted from first light source unit 32 and at least part of the electromagnetic wave emitted from second light source unit 33 when first light source unit 32 and second light source unit 33 are caused to emit the respective electromagnetic waves, so that the electromagnetic waves emitted from first light source unit 32 and the electromagnetic wave emitted from second light source unit 33 can be taken out from the same output aperture (output aperture 31). As a result, it is possible to irradiate more spots of the skin with light (electromagnetic waves) each having a different wavelength spectrum at the same time, compared with those conventionally having been possible before, and to treat with light (electromagnetic waves) exerting a plurality of aesthetic effects at the same time. Therefore, the aesthetic effects can be further enhanced.

Light-emitting unit 30 according to the present exemplary embodiment may further include an optical system disposed between first and second light source units 32, 33 and output aperture 31, and guiding at least part of the electromagnetic waves emitted from at least one of first light source unit 32 and second light source unit 33, to output aperture 31.

With this configuration, the light (electromagnetic waves) emitted from at least one of first light source unit 32 and second light source unit 33 can be guided to the output aperture more efficiently, and the aesthetic effects can be enhanced further.

The optical system may be integrator optical system 34.

With this configuration, light (electromagnetic waves) from light sources having respectively different wavelength spectra can be emitted from the output aperture in a more uniformly mixed manner. As a result, light (electromagnetic waves) from light sources each having a different wavelength spectrum can be applied to the skin more uniformly, and the aesthetic effects on the skin can be enhanced more efficiently.

Integrator optical system 34 may be light pipe 341 surrounded by mirror surface 341a capable of reflecting the electromagnetic waves having the wavelength spectra emitted from first light source unit 32 and second light source unit 33, respectively.

With this configuration, integrator optical system 34 can be built using a simpler configuration.

Mirror surface 341a of light pipe 341 may have a mirror surface inclined with respect to the central axis of light pipe 341.

With this configuration, the light (electromagnetic waves) emitted from at least one of first light source unit 32 and second light source unit 33 can be guided to the output aperture more efficiently, and the aesthetic effects can be enhanced further.

In particular, by using tapered light pipe 341 as described in the present exemplary embodiment, more uniform light (electromagnetic wave) can be delivered to output aperture 31 more efficiently.

Mirror surface 341a of light pipe 341 may have a mirror surface extending in parallel with the central axis of light pipe 341.

With this, mirror surface 341a of light pipe 341 can be provided more easily.

First light source unit 32 may include flash lamp 321 as the first light source, and may include reflection mirror 322 for guiding at least part of the electromagnetic waves emitted from first light source 321 and travelling in a direction other than the direction toward output aperture 31, to output aperture 31.

In this manner, light (electromagnetic waves) emitted from flash lamp 321 can be guided to output aperture 31 more efficiently.

Second light source unit 33 may include LEDs 331 as the second light source.

In this manner, various aesthetic effects can be achieved more easily.

The wavelength spectrum of the electromagnetic waves emitted from LEDs 331 may be temperature-dependent.

Thus, a plurality of aesthetic effects can be achieved only by using one LED.

First light source unit 32 may be disposed not overlapping with the electromagnetic waves emitted in the principal direction from second light source unit 33, until the electromagnetic waves emitted from second light source unit 33 in the principal direction reach output aperture 31; and second light source unit 33 may be disposed not overlapping with the electromagnetic waves emitted in the principal direction from first light source unit 32, until the electromagnetic waves emitted in the principal direction from first light source unit 32 reach output aperture 31.

In this manner, the light (electromagnetic waves) emitted from each of the light source units can be guided to output aperture 31 more efficiently.

Second light source unit 33 may be disposed not overlapping with first light source unit 32 in a view from output aperture 31.

With this, too, it becomes possible to guide the light (electromagnetic waves) emitted from each of the light source units to output aperture 31 without wasting.

Light-emitting surface 32a of first light source unit 32 and light-emitting surface 33a of second light source unit 33 may be substantially on the same plane.

With this, too, it becomes possible to guide the light (electromagnetic waves) emitted from each of the light source units to output aperture 31 without wasting.

First light source unit 32 may be disposed at a position that does not interfere with the electromagnetic waves within the half-value angle of the electromagnetic wave emitted from second light source unit 33, and second light source unit 33 may be disposed at a position that does not interfere with the electromagnetic waves within the half-value angle of the electromagnetic wave emitted from first light source unit 32.

With this, too, it becomes possible to guide the light (electromagnetic waves) emitted from each of the light source units to output aperture 31 without wasting.

Light-emitting beauty device 1 described above in the exemplary embodiment includes light-emitting unit 30.

In this manner, it is possible to achieve a light-emitting beauty device that includes the light-emitting unit capable of further enhancing the aesthetic effects.

Light-emitting beauty device 1 may include attachment 20 including light-emitting unit 30 and main body 10 to which attachment 20 is attached detachably.

In this manner, the attachment including the light-emitting unit can be selected as one of the attachments having various functions. Therefore, a more versatile light-emitting beauty device can be achieved. In addition, the light-emitting unit is made more easily replaceable.

As described above, according to the present exemplary embodiment, it is possible to obtain the light-emitting unit and the light-emitting beauty device capable of further enhancing the aesthetic effects.

[Others] Although the light-emitting unit and the light-emitting beauty device according to the present disclosure have been described in detail above, these descriptions are not intended to limit the scope of the present disclosure, and it is clear to those skilled in the art that various modifications and improvements thereof are still possible.

For example, in the above exemplary embodiment, flash lamp unit 32 and LED unit 33 are disposed in such a manner that light-emitting surface 32a of flash lamp unit 32 and light-emitting surface 33a of LED unit 33 are substantially on the same plane. However, the arrangement of flash lamp unit 32 and LED unit 33 is not limited thereto, and it is sufficient that each of flash lamp unit 32 and LED unit 33 is disposed at positions reducing interference with the electromagnetic waves emitted from the light source unit of the other. For example, light-emitting surface 32a of flash lamp unit 32 and light-emitting surface 33a of LED unit 33 may be provided at different heights, or the principal direction of the electromagnetic waves (light) emitted from flash lamp unit 32 may be set differently from the principal direction of the electromagnetic waves (light) emitted from LED unit 33.

It is also possible to use any other method for preventing interference than that using a positional relationship, e.g., by controlling the behavior of light by disposing an optical system such as a mirror or a lens.

In the above exemplary embodiment, tapered light pipe 341 is used as integrator optical system 34. However, light pipe 341 may be any pipe as long as light can be guided to output aperture 31 more efficiently, as compared with a configuration without any of such an element. For example, an integrator optical system such as a linear light pipe or a fly-eye lens may be used. With such a configuration, it becomes possible to guide the electromagnetic waves (light) to output aperture 31 more efficiently, and to achieve a substantially uniform illuminance distribution in output aperture 31.

In the above exemplary embodiment, flash lamp 32 is used as an example of the first light source, and LEDs 331 are used as the second light source. However, it is only necessary for the first light source and the second light source to be capable of emitting electromagnetic waves, and a laser diode (LD), or an organic electro-luminescence (EL), for example, may also be used. With such a configuration, too, it becomes possible to select a wavelength depending on the aesthetic effects to be achieved, and to achieve desired aesthetic effects and the hair removal effect.

In the above exemplary embodiment, attachment 20 detachably attached to main body 10 is provided, as one example. However, an integrated light-emitting beauty device (a light-emitting beauty device with a non-detachable light-emitting unit) may also be used.

Specifications (e.g., shape, size, or layout) of the main body, the attachment, and other details may also be changed as appropriate.

### INDUSTRIAL APPLICABILITY

As described above, because the light-emitting unit and the light-emitting beauty device according to the present disclosure can further enhance the aesthetic effects, the light-emitting unit and the light-emitting beauty device may be applied to any device achieving some biological effect by irradiating skin with light, specific examples of which include a facial treatment device, a hair removing device, a physical therapy device, and a medical device.

### REFERENCE MARKS IN THE DRAWINGS

- 1: light-emitting beauty device
- 10: main body
- 20: attachment
- 30: light-emitting unit
- 31: output aperture
- 32: flash lamp unit (first light source unit)
- 32a: light-emitting surface
- 321: flash lamp (first light source)
- 322: reflection mirror
- 33: LED unit (second light source unit)
- 33a: light-emitting surface
- 331: LED (second light source)
- 34: integrator optical system (optical system)
- 341: light pipe
- 341a: mirror surface

## Claims

1. A light-emitting unit comprising:
a first light source unit having a predetermined wavelength spectrum;
a second light source unit having a wavelength spectrum different from the predetermined wavelength spectrum of the first light source unit; and
an output aperture through which at least part of a first electromagnetic wave emitted from the first light source unit and at least part of a second electromagnetic wave emitted from the second light source unit are outputted when the first light source unit and the second light source unit are caused to emit the first electromagnetic wave and the second electromagnetic wave, respectively,
wherein the first light source unit is disposed at a position reducing interference with the second electromagnetic wave emitted from the second light source unit, and
the second light source unit is disposed at a position reducing interference with the first electromagnetic wave emitted from the first light source unit.

2. The light-emitting unit according to Claim 1, further comprising an optical system that is disposed between the first and the second light source units and the output aperture, and that guides at least part of at least one of the first electromagnetic wave and the second electromagnetic wave to the output aperture.

3. The light-emitting unit according to Claim 2, wherein the optical system is an integrator optical system.

4. The light-emitting unit according to Claim 3, wherein the integrator optical system is a light pipe surrounded by a mirror surface capable of reflecting the first electromagnetic wave emitted at the predetermined wavelength spectrum of the first light source unit and the second electromagnetic wave emitted at the wavelength spectrum of the second light source unit.

5. The light-emitting unit according to Claim 4, wherein the mirror surface of the light pipe includes a mirror surface inclined with respect to a central axis of the light pipe.

6. The light-emitting unit according to Claim 4 or 5, wherein the mirror surface of the light pipe has a mirror surface extending in parallel with the central axis of the light pipe.

7. The light-emitting unit according to any one of Claims 1 to 6, wherein the first light source unit includes
a flash lamp as a first light source, and
a reflection mirror configured to guide at least part of the first electromagnetic wave emitted from the first light source and travelling in a direction other than a direction toward the output aperture, to the output aperture.

8. The light-emitting unit according to any one of Claims 1 to 7, wherein the second light source unit includes a light emitting diode as a second light source.

9. The light-emitting unit according to Claim 8, wherein the wavelength spectrum of the second electromagnetic wave emitted from the light emitting diode is temperature dependent.

10. The light-emitting unit according to any one of Claims 1 to 9, wherein
the first light source unit is disposed not overlapping with the second electromagnetic wave emitted in a principal direction from the second light source unit, until the second electromagnetic wave emitted in the principal direction from the second light source unit reaches the output aperture, and
the second light source unit is disposed not overlapping with the first electromagnetic wave emitted in a principal direction from the first light source unit, until the first electromagnetic wave emitted in the principal direction from the first light source unit reaches the output aperture.

11. The light-emitting unit according to any one of Claims 1 to 10, wherein the second light source unit is disposed not overlapping with the first light source unit in a view from the output aperture.

12. The light-emitting unit according to any one of Claims 1 to 11, wherein a light-emitting surface of the first light source unit and a light-emitting surface of the second light source unit are substantially on a same plane.

13. The light-emitting unit according to any one of Claims 1 to 12, wherein
the first light source unit is disposed at a position not interfering with an electromagnetic wave within a half-value angle of the second electromagnetic wave emitted from the second light source unit, and
the second light source unit is disposed at a position not interfering with an electromagnetic wave within a half-value angle of the first electromagnetic wave emitted from the first light source unit.

14. A light-emitting beauty device comprising the light-emitting unit according to any one of Claims 1 to 12.

15. The light-emitting beauty device according to Claim 14, further comprising:
an attachment including the light-emitting unit; and
a main body to which the attachment is detachably attached.
